# EUROPEAN PATENT APPLICATION

(11) **EP 2 096 865 A2**
(43) Date of publication of application: **02.09.2009**
(21) Application number: 09002519.8
(22) Date of filing: 23.02.2009
(51) Int. Cl.: H04N 5/335, H03L 7/08, H04L 7/00, H04L 25/49

(54) **Image pickup system and endoscope system**

(30) Priority: 26.02.2008 JP 2008043825
(71) Applicant: FUJIFILM Corporation, Minato-ku Tokyo (JP)
(72) Inventor: Nakamura, Kazuhiko, Saitama (JP)
(74) Representative: Vierheilig, Achim

(57) **Abstract**

An image pickup system includes an image pickup unit (42) having a solid-state image pickup device (44), for example, CMOS. A processor (11) controls the image pickup device and receives an image signal (SDT) from the image pickup unit with a signal line (49) in serial transmission. The processor includes a clock and data recovery circuit (79) for deriving a clock signal (RCLK) from the image signal input by the signal line, and for producing a data signal (RSDT) synchronized with the clock signal in phase synchronization. There is a signal processing unit (81-83) for signal processing according to the data signal and the clock signal produced by the clock and data recovery circuit. Preferably, the image pickup unit includes an A/D converter for digitally converting the image signal from the image pickup device into parallel data of bits of a predetermined number.

## Description

### BACKGROUND OF THE INVENTION

### 1. Field of the Invention

The present invention relates to an image pickup system and endoscope system. More particularly, the present invention relates to an image pickup system and endoscope system in which an image signal can be serially transmitted at a high speed and stably.

### 2. Description Related to the Prior Art

An endoscope system in the field of medical instruments is provided with a solid-state image pickup device of a small size, such as a CCD (Charge Coupled Device) and CMOS (Complementary Metal Oxide Semiconductor). The endoscope system includes an endoscope with an image pickup unit at a head assembly, and a processor or external controller. The endoscope is swallowed orally in a body of a patient, and picks up an image of an object in a gastrointestinal tract. The processor controls operation of the endoscope, and creates an image according to an image signal from the endoscope. There are signal lines for connecting the endoscope with the processor. The image signal is transmitted by the signal lines.

In the endoscope system of a known structure, the image signal from the image pickup device is transmitted to the processor as an analog signal, and is converted digitally in the processor. However, there occurs influence of electric noise to the analog signal according to a great length of the signal lines, to lower the quality of an image created by the processor. There is a suggestion in JP-A 2002-065601 in which the image signal is converted into a digital form in the endoscope with the image pickup unit, and then is transmitted to the processor by the signal lines after the conversion.

In the endoscope system of JP-A 2002-065601, the image signal is converted into a digital form or parallel data of plural bits. The parallel data is converted by the endoscope with the image pickup unit into serial data, which is transmitted by one bit in a time sequential manner. This is effective in reducing the number of the signal lines and raising the characteristic in the transmission.

In the endoscope system of JP-A 2002-065601 with serial transmission of the image signal, the processor on the receiving side detects the image signal in a time sequential manner in the unit of bit. In the processor, recognition of transmission frequency is required for the image signal output by the endoscope with the image pickup unit. If the transmission frequency is low, the processor can recognize the transmission frequency by use of an internal clock signal generated in the processor. However, if the transmission frequency is high, for example over 1 GHz, a difference occurs between the transmission frequency of the image signal and the internal clock signal of the processor, to create an error in the recognition.

To solve such a problem, it is possible to use the technique of high-speed serial communication, in which a clock signal line extends for connection between the endoscope with the image pickup unit and the processor for transmitting a clock signal in addition to a data signal line for transmitting a data signal. The clock signal is transmitted together with the data signal in a synchronized manner. On a receiving side, data is detected according to the transmitted clock signal.

However, the endoscope system has a feature of a great distance between the endoscope with the image pickup unit for transmission and the processor for reception, and has the signal lines of a great length. Even when the high-speed serial communication is used, there occurs a timing skew or phase difference between a data signal and clock signal on the data signal line and the clock signal line for the reason of capacity and resistance of wires of the signal lines. Transmission of the image signal may be unstable, to cause errors in detecting data on the receiving side. Degradation of images, errors in the display and other failure may occur with the occurrence in errors in detecting data.

### SUMMARY OF THE INVENTION

In view of the foregoing problems, an object of the present invention is to provide an image pickup system and endoscope system in which an image signal can be serially transmitted at a high speed and stably.

In order to achieve the above and other objects and advantages of this invention, an image pickup system includes an image pickup unit having a solid-state image pickup device. A processor controls the solid-state image pickup device and for receiving an image signal from the image pickup unit with a signal line in serial transmission. The processor includes a clock and data recovery circuit for deriving a clock signal from the image signal input by the signal line, and for producing a data signal synchronized with the clock signal in phase synchronization. There is a signal processing unit for signal processing according to the data signal and the clock signal produced by the clock and data recovery circuit.

The solid-state image pickup device is a CMOS image sensor.

The image pickup unit includes an A/D converter for digitally converting the image signal from the solid-state image pickup device into parallel data of bits of a predetermined number.

The image pickup unit includes a parallel/serial converter for converting the parallel data from the A/D converter into serial data, and the serial data is transmitted with the signal line.

The image pickup unit includes an encoder for encoding the image signal to set a higher number of bits in the image signal, for preventing a signal level from remaining equal in the serial data in a predetermined period or more.

The clock and data recovery circuit includes a voltage control oscillator, supplied with a signal of control, for generating a clock signal at a variable frequency of oscillation. A phase comparator is supplied with the clock signal and serial data of the image signal, for producing and applying a phase difference signal to the voltage control oscillator, to produce the clock signal at an adjusted frequency according to the serial data of the image signal.

The clock and data recovery circuit further includes a flip-flop, supplied with the serial data, for producing the data signal by sampling according to the clock signal from the voltage control oscillator.

The image pickup unit includes a differential signal transmitter for transmitting a differential signal according to the image signal. The processor includes a differential signal receiver, connected with the differential signal transmitter, for receiving the differential signal in the serial transmission.

The signal processing unit includes a serial/parallel converter for converting the data signal into parallel data. A decoder converts the parallel data into the image signal. An image processing circuit converts the image signal into image data.

The image pickup unit includes an 8B10B encoder for converting the image signal of 8 bits into the data signal of 10 bits for the serial transmission. The decoder is an 8B10B decoder for converting the data signal of 10 bits into the image signal of 8 bits.

The signal processing unit includes a PLL circuit for changing frequency of a clock signal for signal processing.

In one aspect of the invention, an endoscope system includes an electronic endoscope having a solid-state image pickup device. A processor controls the solid-state image pickup device and for receiving an image signal from the endoscope with a signal line in serial transmission. The processor includes a clock and data recovery circuit for deriving a clock signal from the image signal input by the signal line, and for producing a data signal synchronized with the clock signal in phase synchronization. There is a signal processing unit for signal processing according to the data signal and the clock signal produced by the clock and data recovery circuit.

Accordingly, an image signal can be serially transmitted at a high speed and stably from an image pickup unit to a processor or external controller.

### BRIEF DESCRIPTION OF THE DRAWINGS

The above objects and advantages of the present invention will become more apparent from the following detailed description when read in connection with the accompanying drawings, in which:
Fig. 1 is an explanatory view illustrating an endoscope system;
Fig. 2 is a front elevation illustrating a front surface of an endoscope;
Fig. 3 is a vertical section partially broken, illustrating a head assembly of the endoscope;
Fig. 4 is a block diagram illustrating an image pickup device;
Fig. 5 is a block diagram illustrating the image pickup device and a processor or external controller;
Fig. 6 is an explanatory view illustrating a parallel/serial conversion of pixel data;
Fig. 7 is a block diagram illustrating a clock and data recovery circuit;
Fig. 8 is a timing chart illustrating the operation of the clock and data recovery circuit; and
Fig. 9 is a block diagram illustrating an example according to LVDS (Low Voltage Differential Signaling) transmission.

### DETAILED DESCRIPTION OF THE PREFERRED

### EMBODIMENT(S) OF THE PRESENT INVENTION

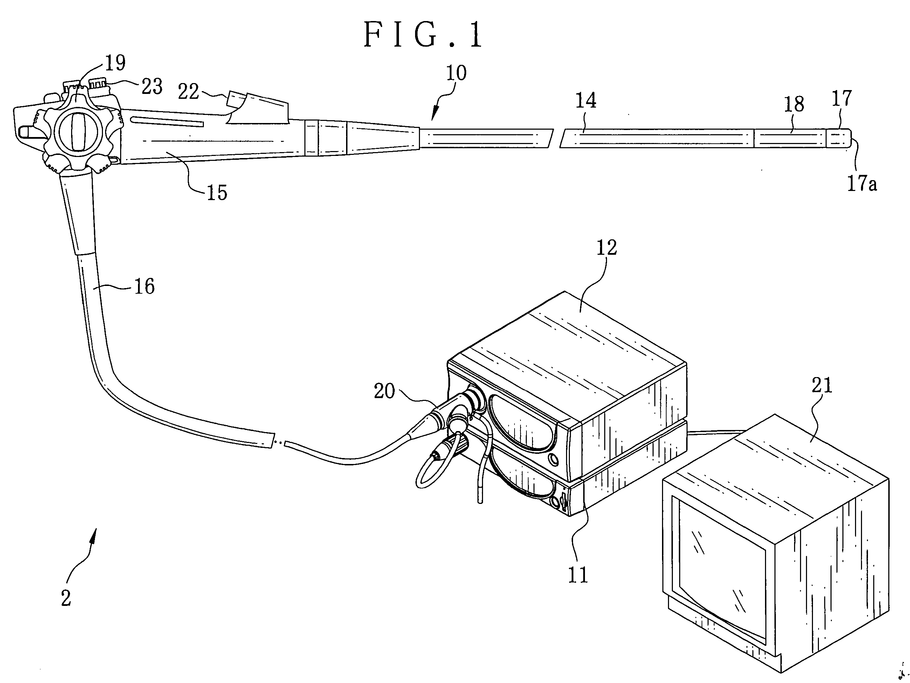

In Fig. 1, an endoscope system 2 includes an electronic endoscope 10, a processor or external controller 11 and a light source 12. The endoscope 10 includes an insertion tube 14, a handle 15, and a universal cord 16. The insertion tube 14 is flexible and inserted in a gastrointestinal tract of a patient' s body. The handle 15 extends from the proximal end of the insertion tube 14. The universal cord 16 is connected between the processor 11 and the light source 12.

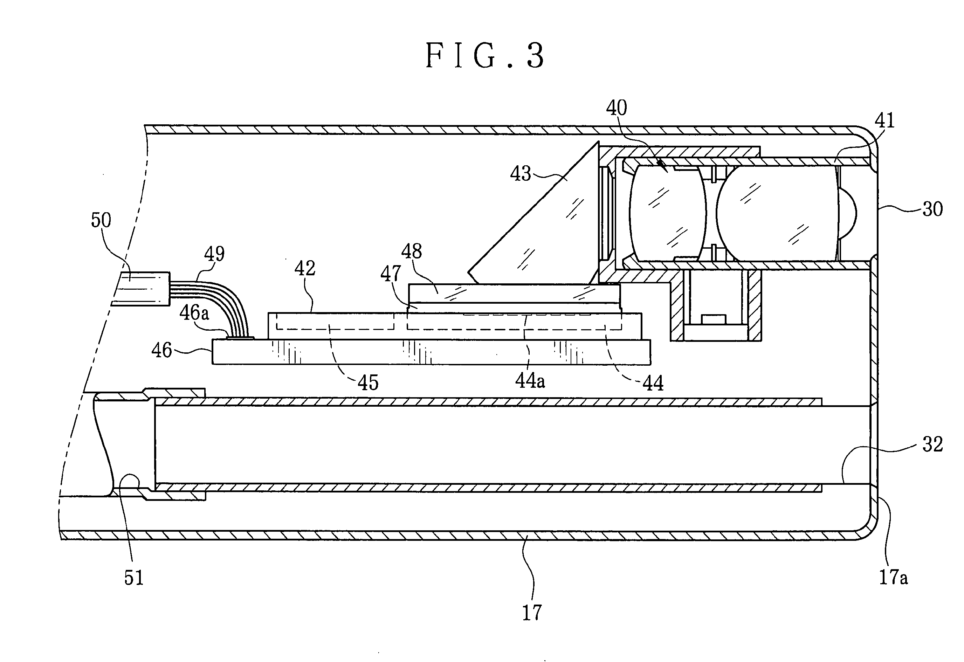

There is a head assembly 17 on the insertion tube 14. An image pickup unit 42 of Fig. 3 is incorporated in the head assembly 17 for in-vivo imaging. A steering portion 18 extends from a proximal end of the head assembly 17, and includes plural curved segments. A steering wheel 19 or angle knob is disposed on the handle 15, and is manually rotated to move a wire back and forth through the insertion tube 14, to bend the steering portion 18 up and down and to the right and left. Thus, the head assembly 17 can tilt in the body in any intended direction.

A proximal end of the universal cord 16 is connected with a connector 20. The connector 20 is a composite type, and used for connection with the processor 11 and also the light source 12.

A cable 50 extends through the universal cord 16 as illustrated in Fig. 3. The processor 11 supplies the endoscope 10 with power, and controls operation of the image pickup unit 42, and receives an image signal transmitted from the image pickup unit 42 by the cable 50, to convert the received image signal into image data by signal processing of various types. A monitor display panel 21 displays an endoscopic image in connection with the processor 11 according to the converted image data. Also, the processor 11 is connected with the light source 12 electrically by the connector 20, and controls the entirety of the endoscope system 2.

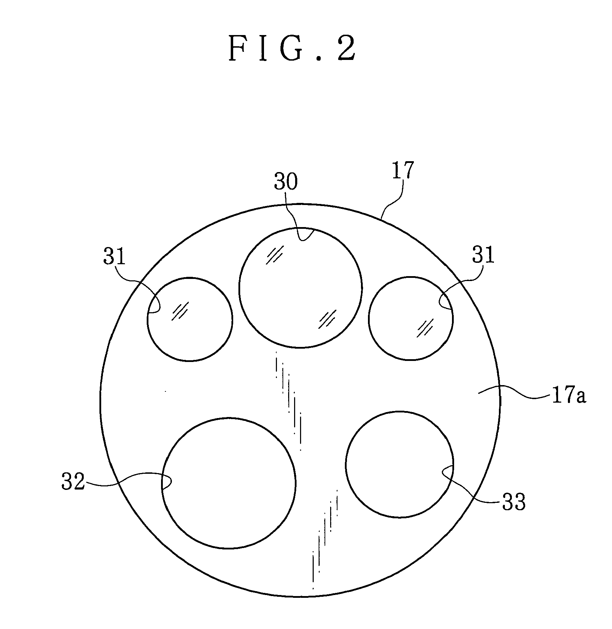

In Fig. 2, the head assembly 17 has a front end surface 17a. An imaging window 30, lighting openings 31, a first forceps opening 32 and an air/water nozzle 33 are formed in the front end surface 17a. The imaging window 30 is disposed at the center of the head assembly 17. The lighting openings 31 are disposed symmetrically with respect to the imaging window 30, and apply light from the light source 12 to an object in the gastrointestinal tract. A forceps channel 51 of Fig. 3 is formed to extend in the insertion tube 14 toward the first forceps opening 32. A second forceps opening 22 is formed in the handle 15, and is open at an end of the forceps channel 51. A medical instrument or tool having an injection needle, high frequency incision device or the like is inserted in the second forceps opening 22. A distal end of the medial instrument appears through the first forceps opening 32. The air/water nozzle 33 emits washing water or air from an air/water supply device in the light source 12 toward an object in the body or to the imaging window 30 when an air/water supply button 23 on the handle 15 of Fig. 1 is manually operated.

In Fig. 3, a barrel 41 is positioned on an inner side from the imaging window 30. An objective optical system 40 is mounted in the barrel 41 for receiving image light from an object in the body. The barrel 41 is so directed that an optical axis of the objective optical system 40 extends in parallel with the center axis of the insertion tube 14. A prism 43 is coupled with the proximal end of the barrel 41, and directs image light of an object from the objective optical system 40 toward the image pickup unit 42 by bending substantially vertically.

The image pickup unit 42 is a CMOS image sensor or monolithic semiconductor chip, and includes a CMOS image pickup device 44 and a peripheral circuit 45. The peripheral circuit 45 drives the image pickup device 44, and inputs and outputs signals. There is a board 46 for support on which the image pickup unit 42 is mounted. An image pickup surface 44a of the image pickup device 44 is opposed to an exit surface of the prism 43. A spacer 47 of a quadrilateral shape is disposed on the image pickup surface 44a. A glass cover 48 of a quadrilateral shape is attached to the spacer 47 and covers the image pickup surface 44a. The image pickup unit 42, the spacer 47 and the glass cover 48 are attached by use of adhesive agent. The image pickup surface 44a is protected from entry of dust or the like.

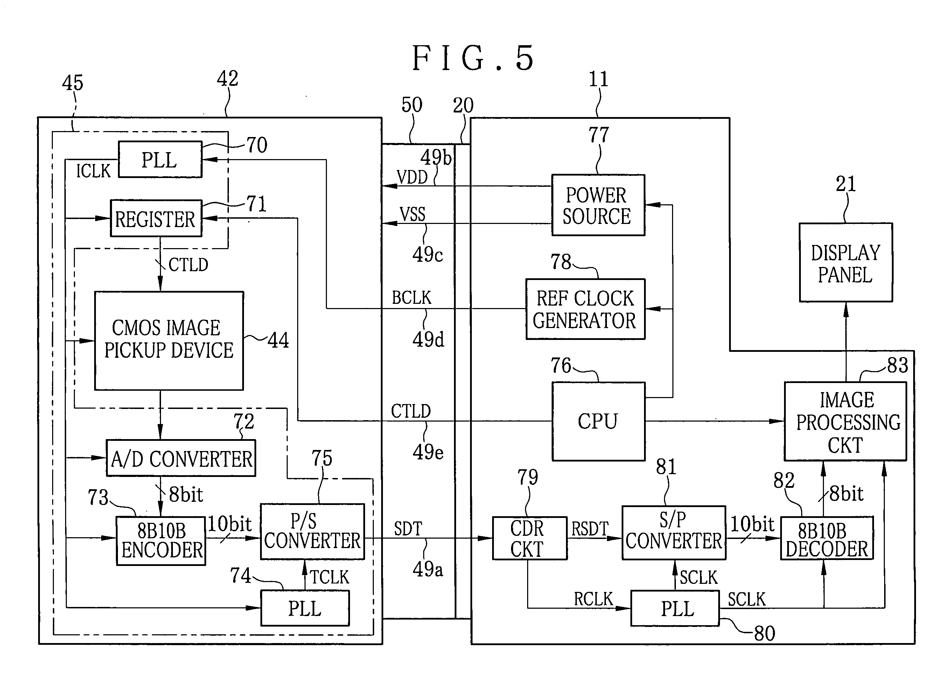

There are plural input/output terminals 46a on a portion of the board 46 extending toward the proximal end in the insertion tube 14. Signal lines 49 (namely signal lines 49a, 49b, 49c, 49d and 49e in Fig. 5) are connected with the input/output terminals 46a with the universal cord 16 for transmitting and receiving various signals with the processor 11. To the peripheral circuit 45 in the image pickup unit 42, the input/output terminals 46a are connected electrically by wires, bonding pads or the like (not shown) positioned on the board 46. In the cable 50, the signal lines 49 are inserted through a flexible tube. The cable 50 extends through the insertion tube 14, the handle 15 and the universal cord 16, and is connected with the connector 20.

An illuminator (not shown) is disposed on an inner side from the lighting openings 31. An exit end of a light guide is disposed at the illuminator and guides light from the light source 12. The light guide extends through the insertion tube 14, the handle 15 and the universal cord 16 in a manner similar to the cable 50. An entrance end of the light guide is coupled with the connector 20.

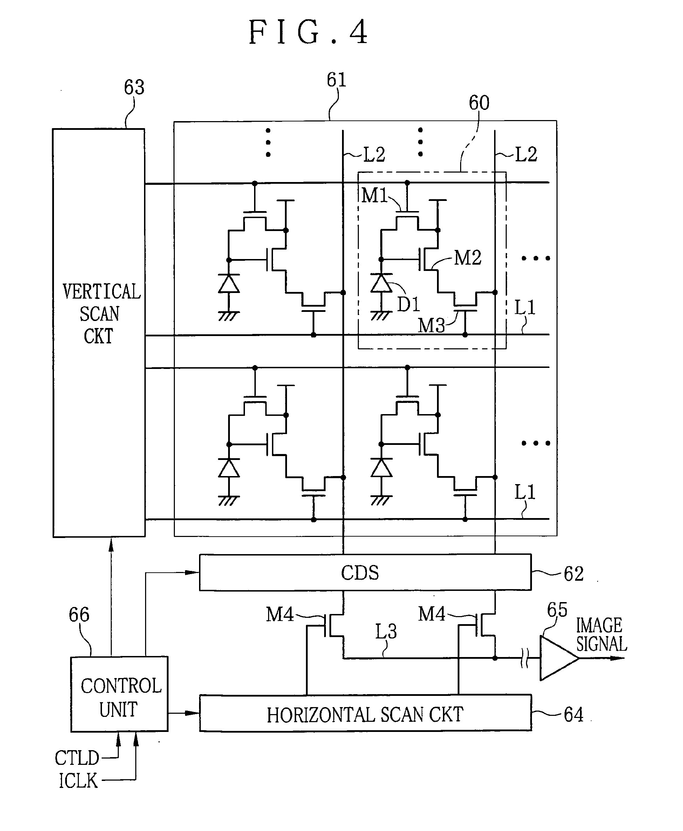

In Fig. 4, the image pickup device 44 includes a pixel array 61, a correlated double sampling circuit 62 or CDS, a vertical scan circuit 63, a horizontal scan circuit 64, an output circuit 65, and a control unit 66. The pixel array 61 includes unit pixels or active pixel cells 60 arranged in a matrix form. The CDS 62 processes pixel data output by the pixel array 61 for noise reduction. The vertical scan circuit 63 controls the scan in the vertical direction and controls the resetting of the pixel array 61. The horizontal scan circuit 64 controls the scan in the horizontal direction. The output circuit 65 outputs pixel data. The control unit 66 sends a control signal to the CDS 62 and the vertical and horizontal scan circuits 63 and 64 to control a time sequence for the vertical and horizontal scan and sampling and the like.

Each of the active pixel cells 60 includes a single photo diode D1, a reset transistor M1, a driving transistor M2 for amplification, and a transistor M3 for selecting pixels. The active pixel cell 60 is connected with a vertical scan line (row selection line) L1 and a horizontal scan line (column signal line) L2, and is scanned by the vertical and horizontal scan circuits 63 and 64 successively.

The control unit 66 generates a control signal for the vertical and horizontal scan circuits 63 and 64 to scan rows and columns in the pixel array 61, a control signal for the vertical scan circuit 63 to reset the signal charge stored in the photo diode D1, and a control signal for the CDS 62 to control connection between the pixel array 61 and the CDS 62.

The CDS 62 is disposed respectively for a column signal line L2 in a split manner, and outputs pixel data of the active pixel cell 60 connected with a row selection line L1 selected by the vertical scan circuit 63 successively with a horizontal scan signal output by the horizontal scan circuit 64. The horizontal scan circuit 64 controls the conductive and non-conductive state of the transistor M4 according to the horizontal scan signal, the transistor being connected between the CDS 62 and the output bus line L3 in connection with the output circuit 65. The output circuit 65 amplifies and outputs pixel data transferred from the CDS 62 to the output bus line L3 successively. A term of "image signal" is hereinafter used for a series of pixel data output by the output circuit 65.

The image pickup device 44 is a single-chip device for color imaging, and includes color filters of plural color segments, for example, color filters of primary colors in the Bayer arrangement.

In Fig. 5, the peripheral circuit 45 in the image pickup unit 42 includes a PLL circuit 70 or phase locked loop circuit, a register 71, an A/D converter 72, an 8B10B encoder 73, a PLL circuit 74 and a parallel/serial converter 75 or P/S converter. The PLL circuit 70 produces an internal clock signal. The register 71 sets control data in the image pickup device 44. The A/D converter 72 digitally converts the image signal from the image pickup device 44. The 8B10B encoder 73 encodes the digital image signal according to 8B10B encoding. The PLL circuit 74 multiplies frequency of the internal clock signal to produce a clock signal for the serial transmission. The parallel/serial converter 75 converts the encoded image signal into serial data.

The PLL circuit 70 is a phase synchronizing circuit, and includes a phase comparator, loop filter, voltage control oscillator and prescaler, is synchronized with a reference clock signal BCLK input stably by the processor 11, and produces an inner clock signal ICLK having a frequency which is proportional to a frequency of the reference clock signal BCLK. The inner clock signal ICLK is supplied to relevant elements of the peripheral circuit 45 and the control unit 66 in the image pickup device 44. See Fig. 4.

The register 71 retains the control data CTLD which is from the processor 11 to drive the image pickup device 44, and inputs the control data to the control unit 66 of Fig. 4. The register 71 is a shift register for the serial/parallel conversion, and converts the control data CTLD of a serial form into parallel data, which is input to the control unit 66. Examples of information represented by the control data CTLD include a scanning type of pixels (full frame scan or interlace scan), a pixel location for scan (position of the active pixel cell 60 for start and end of scan), and a shutter speed (exposure time). The control unit 66 controls the CDS 62 and the vertical and horizontal scan circuits 63 and 64 in the image pickup device 44 according to the control data CTLD and internal clock signal ICLK.

The A/D converter 72 converts an image signal from the image pickup device 44 into a digital signal of 8 bits (256 steps of gradation) by quantizing pixel data as an analog signal. The digital signal of 8 bits is input to the 8B10B encoder 73 in parallel by use of eight lines.

The 8B10B encoder 73 according to 8B10B encoding converts pixel data of 8 bits from the A/D converter 72 into pixel data of 10 bits by addition of data of 2 bits. A standardized conversion table is used for the 8B10B encoding. The conversion is for the purpose of preventing continuity of an equal signal level of 0 or 1 for a predetermined period or more at the time of the serial transmission which will be described later. If the pixel data of 8 bits is "00000000", the pixel data is converted into data of 10 bits of "1001110100". If the pixel data of 8 bits is "00001111", the pixel data is converted into data of 10 bits of "0101110100".

The PLL circuit 74 is structurally equal to the PLL circuit 70, produces a clock signal TCLK for serial transmission by multiplication of 10 times as high as the frequency of the internal clock signal ICLK, and supplies the parallel/serial converter 75 with the clock signal TCLK.

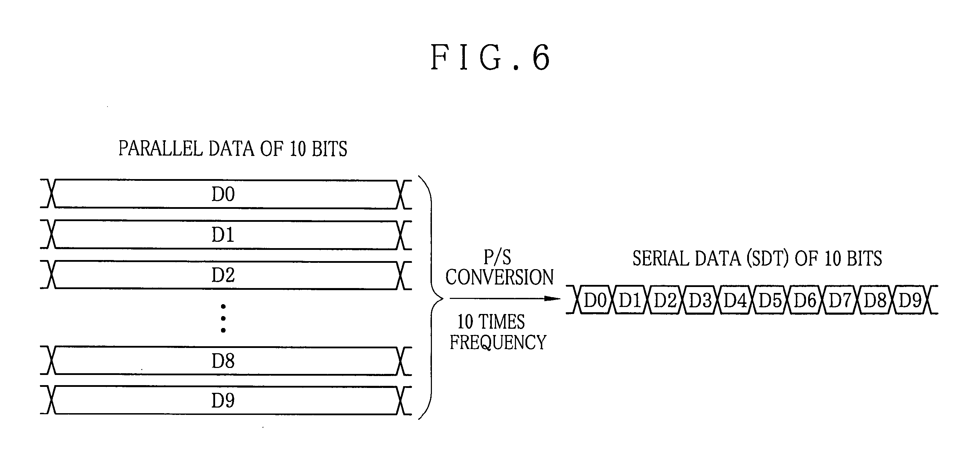

The parallel/serial converter 75 responds to the serial transmission clock signal TCLK from the PLL circuit 74, and converts pixel data or parallel data of 10 bits from the 8B10B encoder 73 into serial data of 10 bits. See Fig. 6. The frequency of the converted serial data is set 10 times as high by the PLL circuit 74 as that of the parallel data before the conversion. The serial data created by the parallel/serial converter 75 is transmitted to the processor 11 by the signal line 49a of the cable 50 by way of the image signal SDT.

The processor 11 includes a CPU 76 as main control unit, a power source 77, a reference clock generator 78, a clock and data recovery circuit 79 or CDR circuit, a PLL circuit 80, a serial/parallel converter 81 or S/P converter, an 8B10B decoder 82, and an image processing circuit 83. The CPU 76 controls the entirety of elements in the processor 11. The power source 77 generates a power source voltage VDD and a grounded voltage or grounded potential VSS. The reference clock generator 78 generates a reference clock signal BCLK. The clock and data recovery circuit 79 receives an image signal SDT from the image pickup unit 42, and recovers a clock signal and data signal responsively. The PLL circuit 80 multiplies frequency of the clock signal generated by the clock and data recovery circuit 79, and generates a clock signal for signal processing with a frequency equal to that of the internal clock signal ICLK in the image pickup unit 42. The serial/parallel converter 81 converts the data signal from the clock and data recovery circuit 79 into parallel data. The 8B10B decoder 82 decodes the parallel data of the image according to 8B10B decoding, to produce the image signal before encoding. The image processing circuit 83 processes the decoded image signal by image processing, to produce image data of an image to be displayed on the display panel 21. According to the embodiment, a signal processing unit as a feature of the scope of the invention is constituted by the PLL circuit 80, the serial/parallel converter 81, the 8B10B decoder 82 and the image processing circuit 83.

The power source 77 supplies various elements in the processor 11 with the power source voltage VDD and a grounded voltage or grounded potential VSS, and also supplies the image pickup unit 42 with the same by use of the signal lines 49b and 49c. The reference clock generator 78 generates a reference clock signal BCLK with the stable frequency, and sends this to the PLL circuit 70 in the image pickup unit 42 by use of the signal line 49d.

The CPU 76 controls relevant elements in the processor 11, produces the control data CTLD described above, and supplies the register 71 in the image pickup unit 42 with the control data CTLD through the signal line 49e.

The clock and data recovery circuit 79 detects a phase of the image signal SDT transmitted serially from the image pickup unit 42, generates a derived clock signal RCLK synchronized with the frequency of the image signal SDT, and creates the image signal RSDT or retimed data by sampling the image signal SDT by use of the derived clock signal RCLK. The image signal RSDT or retimed data is constituted by retiming the image signal SDT according to the derived clock signal RCLK.

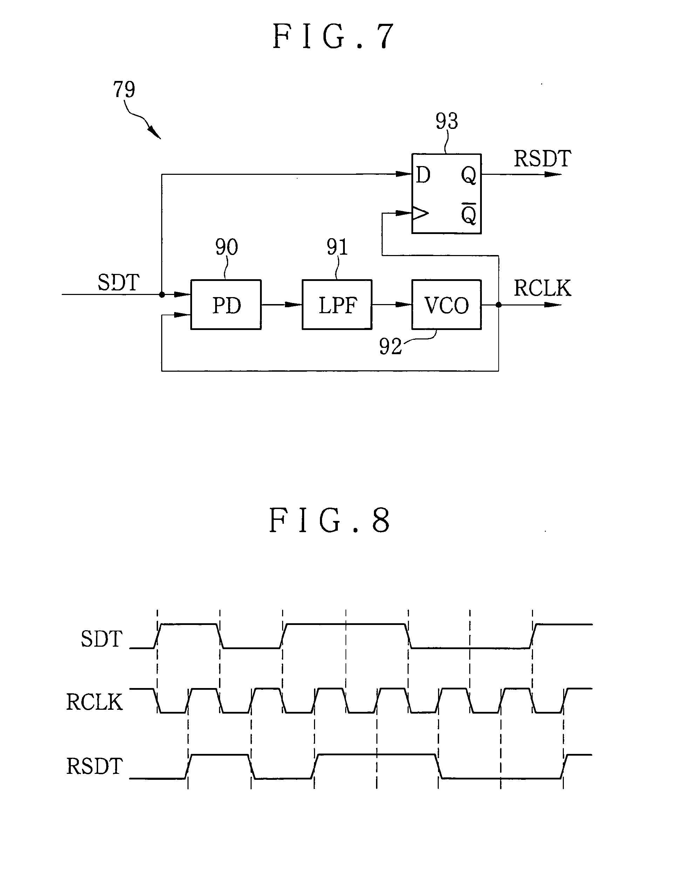

Specifically, the clock and data recovery circuit 79 includes a phase comparator 90 or PD, a loop filter 91 or LPF, and a voltage control oscillator 92 or VCO, and a D type flip-flop 93. See Fig. 7. The phase comparator 90 is supplied with the image signal SDT and the derived clock signal RCLK generated by the VCO 92. An output of the phase comparator 90 is sent to the VCO 92 through the loop filter 91. In the D type flip-flop 93, the image signal SDT is input to the data input terminal D. The derived clock signal RCLK is input to the clock input terminal.

The phase comparator 90 detects a phase difference by comparing rising edges of the image signal SDT and the derived clock signal RCLK, and supplies the VCO 92 with a detection signal by use of the loop filter 91. The VCO 92 responds to the detection signal, and changes the frequency of the derived clock signal RCLK. In Fig. 8, the VCO 92 outputs the derived clock signal RCLK synchronized with the frequency of the image signal SDT.

The D type flip-flop 93 samples the image signal SDT on a rising edge of the derived clock signal RCLK to hold data. The D type flip-flop 93 recovers an image signal RSDT as retimed data in phase synchronization with the derived clock signal RCLK, and outputs the same at the data output terminal Q. The derived clock signal RCLK from the clock and data recovery circuit 79 is input to the PLL circuit 80. The recovered image signal RSDT is input to the serial/parallel converter 81.

In Fig. 5, the PLL circuit 80 is structurally equal to the PLL circuit 70, produces a clock signal SCLK for signal processing by setting 1/10 time as high as the frequency of the derived clock signal RCLK, and supplies the serial/parallel converter 81, the 8B10B decoder 82 and the image processing circuit 83 with the clock signal SCLK. The clock signal SCLK has an equal frequency to that of the internal clock signal ICLK.

The serial/parallel converter 81 responds to the clock signal SCLK from the PLL circuit 80, and converts the image signal RSDT from the clock and data recovery circuit 79 into an image signal of parallel data (pixel data) of 10 bits according to the serial/parallel conversion which is reverse to the parallel/serial conversion of Fig. 6. The image signal of the parallel data is input to the 8B10B decoder 82.

The 8B10B decoder 82 operates according to the conversion table of the standardized 8B10B encoding, and forms data of 8 bits from the input image signal of 10 bits by the conversion reverse to that of the 8B10B encoder 73. The image signal of pixel data of 8 bits recovered by the 8B10B decoder 82 is input to the image processing circuit 83.

The image processing circuit 83 responds to the clock signal SCLK, detects pixel data included in the image signal, writes the pixel data to the internal memory, and produces image data by image processing of white balance adjustment, gain correction, color interpolation, edge enhancement, gamma correction, color matrix operation and the like. The image processing circuit 83 converts image data into a signal format for display on the display panel 21, and causes the display panel 21 to display an image.

For imaging of an object in a gastrointestinal tract with the endoscope system 2, at first the endoscope 10, the processor 11, the light source 12 and the display panel 21 are powered. The insertion tube 14 of the endoscope 10 is swallowed orally in the body. Light from the light source 12 is applied to the object, for a doctor to observe an image of the object from the image pickup device 44 on the display panel 21.

The image signal from the image pickup device 44 is converted into parallel data of 8 bits by the A/D converter 72, then is converted into parallel data of 10 bits by the 8B10B encoder 73. The parallel data of 10 bits as image signal is converted into serial data by the parallel/serial converter 75, and transmitted to the processor 11 by the signal line 49a.

In the processor 11, the clock and data recovery circuit 79 receives the image signal transmitted serially, and generates the derived clock signal RCLK and a data signal or retimed data RSDT synchronized with the clock signal in the phase synchronization. The image signal RSDT created by the clock and data recovery circuit 79 as retimed data is converted by the serial/parallel converter 81 and the 8B10B decoder 82 according to the derived clock signal RCLK, to recover the parallel data of 8 bits. The image signal or the parallel data of 8 bits is converted into image data by the image processing circuit 83, to cause the display panel 21 to display an image.

As described heretofore, the endoscope system 2 transmits the image signal in the serial transmission by use of the signal line 49a from the image pickup unit 42. In the processor 11, the clock and data recovery circuit 79 extracts a clock signal from the transmitted image signal, and also produces a data signal in the phase synchronization with the clock signal. Therefore, there occurs no problem of timing skew between the data signal and the clock signal even in the serial transmission, which can be carried out at a high speed and stably. It is possible in the processor 11 correctly to detect pixel data from the image signal, to prevent degradation of the image, an error in the display and other failure.

In the endoscope system 2, the image signal is transmitted serially with the signal line 49a, so that the endoscope 10 can have a small diameter by reducing the diameter of the cable 50. Physical load to a patient in which the endoscope 10 is swallowed is reduced.

The endoscope system 2 carries out the serial transmission after conversion of the image signal by the 8B10B encoder 73 in a form without continuity of data over a predetermined length of time. The number of times of transition of data can be greater in the period of the transmitted serial data. The frequency of occurrence of the rising edge increases. Therefore, it is possible in the phase comparator 90 of the clock and data recovery circuit 79 to extract a clock signal constantly correctly.

Although the image pickup device 44 is a CMOS type, the solid-state image pickup device 44 in the invention can be a CCD image sensor or the like.

In the above embodiment, pixel data of 8 bits created by the A/D converter 72 is converted by the 8B10B encoder 73 into pixel data of 10 bits. However, the numbers of bits before and after the conversion can be modified suitably according to the invention.

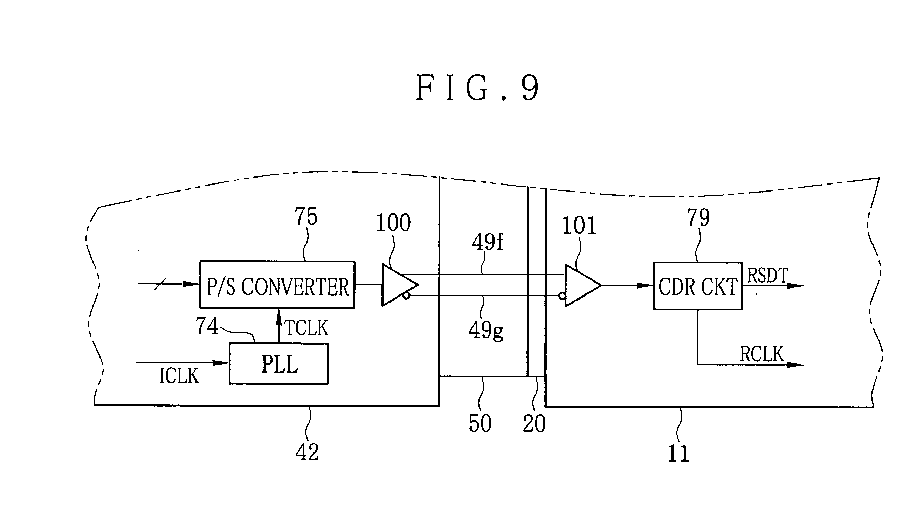

In the above embodiment, the single signal line is used for serial transmission of an image signal. It is possible as illustrated in Fig. 9 to use a differential signal transmitter 100 and a differential signal receiver 101. The differential signal transmitter 100 is connected with the parallel/serial converter 75 on the transmission side. The differential signal receiver 101 is connected with the clock and data recovery circuit 79 on the reception side. Signal lines 49f and 49g extend for connection between the differential signal transmitter 100 and the differential signal receiver 101, for serial transmission of an image signal according to LVDS (Low Voltage Differential Signaling) transmission for serial data as a differential signal. Therefore, it is possible to prevent influence of external electric noise.

In the above embodiment, the image pickup system is an endoscope system. However, an image pickup system may be any suitable one of known systems, such as an ultrasonic endoscope system including an ultrasonic vibration probe, a digital camera including a removable lens assembly, a web camera system including a camera and a personal computer, and the like.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. An image pickup system comprising:
an image pickup unit (42) having a solid-state image pickup device (44);
a processor (11) for controlling said solid-state image pickup device and for receiving an image signal (SDT) from said image pickup unit with a signal line (49) in serial transmission;
said processor including:
a clock and data recovery circuit (79) for deriving a clock signal (RCLK) from said image signal input by said signal line, and for producing a data signal (RSDT) synchronized with said clock signal in phase synchronization; and
a signal processing unit (81-83) for signal processing according to said data signal and said clock signal produced by said clock and data recovery circuit.

2. An image pickup system as defined in claim 1, wherein said solid-state image pickup device is a CMOS image sensor.

3. An image pickup system as defined in claim 1, wherein said image pickup unit includes an A/D converter for digitally converting said image signal from said solid-state image pickup device into parallel data of bits of a predetermined number.

4. An image pickup system as defined in claim 3, wherein said image pickup unit includes a parallel/serial converter for converting said parallel data from said A/D converter into serial data, and said serial data is transmitted with said signal line.

5. An image pickup system as defined in claim 4, wherein said image pickup unit includes an encoder for encoding said image signal to set a higher number of bits in said image signal, for preventing a signal level from remaining equal in said serial data in a predetermined period or more.

6. An image pickup system as defined in claim 1, wherein said clock and data recovery circuit includes:
a voltage control oscillator, supplied with a signal of control, for generating a clock signal at a variable frequency of oscillation;
a phase comparator, supplied with said clock signal and serial data of said image signal, for producing and applying a phase difference signal to said voltage control oscillator, to produce said clock signal at an adjusted frequency according to said serial data of said image signal.

7. An image pickup system as defined in claim 6, wherein said clock and data recovery circuit further includes a flip-flop, supplied with said serial data, for producing said data signal by sampling according to said clock signal from said voltage control oscillator.

8. An image pickup system as defined in claim 1, wherein said image pickup unit includes a differential signal transmitter for transmitting a differential signal according to said image signal;
said processor includes a differential signal receiver, connected with said differential signal transmitter, for receiving said differential signal in said serial transmission.

9. An image pickup system as defined in claim 1, wherein said signal processing unit includes:
a serial/parallel converter for converting said data signal into parallel data;
a decoder for converting said parallel data into said image signal; and
an image processing circuit for converting said image signal into image data.

10. An image pickup system as defined in claim 9, wherein said image pickup unit includes an 8B10B encoder for converting said image signal of 8 bits into said data signal of 10 bits for said serial transmission;
said decoder is an 8B10B decoder for converting said data signal of 10 bits into said image signal of 8 bits.

11. An image pickup system as defined in claim 1, wherein said signal processing unit includes a PLL circuit for changing frequency of a clock signal for signal processing.

12. An endoscope system comprising:
an electronic endoscope (10) having a solid-state image pickup device (44);
a processor (11) for controlling said solid-state image pickup device and for receiving an image signal (SDT) from said endoscope with a signal line (49) in serial transmission;
said processor including:
a clock and data recovery circuit (79) for deriving a clock signal (RCLK) from said image signal input by said signal line, and for producing a data signal (RSDT) synchronized with said clock signal in phase synchronization; and
a signal processing unit (81-83) for signal processing according to said data signal and said clock signal produced by said clock and data recovery circuit.

13. An endoscope system as defined in claim 12, wherein said solid-state image pickup device is a CMOS image sensor.

14. An endoscope system as defined in claim 12, wherein said endoscope includes an A/D converter for digitally converting said image signal from said solid-state image pickup device into parallel data of bits of a predetermined number.

15. An endoscope system as defined in claim 14, wherein said endoscope includes a parallel/serial converter for converting said parallel data from said A/D converter into serial data, and said serial data is transmitted with said signal line.

16. An endoscope system as defined in claim 15, wherein said endoscope includes an encoder for encoding said image signal to set a higher number of bits in said image signal, for preventing a signal level from remaining equal in said serial data in a predetermined period or more.
